# EUROPEAN PATENT SPECIFICATION

(11) **EP 2 356 234 B1**
(45) Date of publication and mention of the grant of the patent: **16.08.2017**
(21) Application number: 09759924.5
(22) Date of filing: 13.11.2009
(51) Int. Cl.: C12N 15/113, A61K 31/7088, A61P 35/00

(54) **DOSAGE OF OLIGONUCLEOTIDES SUITABLE FOR THE TREATMENT OF TUMORS**
DOSIERUNG VON OLIGONUCLEOTIDEN ZUR BEHANDLUNG VON TUMOREN
Dosage d'oligonucléotides adaptés pour le traitement de tumeurs

(30) Priority: 14.11.2008 EP 08169181; 25.03.2009 EP 09156201; 12.05.2009 US 453487
(43) Date of publication of application: 17.08.2011
(73) Proprietor: Autotelic LLC, City of Industry, CA 91748 (US)
(72) Inventor: SCHLINGENSIEPEN, Karl-Hermann, 93093 Donaustauf (DE); HEINRICHS, Hubert, 93059 Regensburg (DE); SCHMAUS, Susanne, 93080 Grossberg-Pentling (DE)
(74) Representative: V.O.
(86) International application number: PCT/EP2009/065179
(87) International publication number: WO 2010/055148

(56) References cited:
- EP-A- 1 008 649
- WO-A-99/63975
- WO-A-2004/104197
- WO-A-2005/059133
- WO-A-2006/117400
- SCHLINGENSIEPEN REIMAR ET AL: "Intracerebral and intrathecal infusion of the TGF-beta 2-specific antisense phosphorothioate oligonucleotide AP 12009 in rabbits and primates: Toxicology and safety" OLIGONUCLEOTIDES, vol. 15, no. 2, 2005, pages 94-104, XP002523633 ISSN: 1545-4576 cited in the application
- SCHLINGENSIEPEN K H ET AL: "Targeted tumor therapy with the TGF-beta2 antisense compound AP 12009" CYTOKINE AND GROWTH FACTOR REVIEWS, vol. 17, no. 1-2, 1 February 2006 (2006-02-01), pages 129-139, XP024987943
- JANSEN B ET AL: "Antisense therapy for cancer-the time of truth" LANCET ONCOLOGY, vol. 3, no. 11, 1 November 2002 (2002-11-01), pages 672-683, XP004810743 ISSN: 1470-2045
- TAMM ET AL: "Antisense therapy in oncology: new hope for an old idea?" LANCET THE, vol. 358, no. 9280, 11 August 2001 (2001-08-11), pages 489-497, XP005018285 ISSN: 0140-6736
- OETTLE H ET AL: "Trabedersen (AP 12009) in the treatment of pancreatic carcinoma and other malignant tumors: interim results of the Phase I/II study" EUROPEAN JOURNAL OF CANCER. SUPPLEMENT, vol. 7, no. 2, 1 September 2009 (2009-09-01), page 138, XP026689278 ISSN: 1359-6349
- SCHLINGENSIEPEN K H ET AL: "Antisense therapeutics for tumor treatment: the TGF-beta2 inhibitor AP 12009 in clinical development against malignant tumors." RECENT RESULTS IN CANCER RESEARCH, vol. 177, 2008, pages 137-150, XP008119612 ISSN: 0080-0015
- [Online] Retrieved from the Internet: <URL:http://ncifrederick.cancer.gov/Lasp/Ac uc/Frederick/Media/Documents/ACUC42.pdf> [retrieved on 2013-03-13]
- FREIREICH E J ET AL: "QUANTITATIVE COMPARISON OF TOXICITY OF ANTICANCER AGENTS IN MOUSE, RAT HAMSTER, DOG, MONKEY, AND MAN", CANCER CHEMOTHERAPY REPORTS, vol. 50, no. 4, 1 May 1966 (1966-05-01), pages 219-244, XP008025817,
- LENNOX K A ET AL: "Chemical modification and design of anti-miRNA oligonucleotides", GENE THERAPY, vol. 18, no. 12, December 2011 (2011-12), pages 1111-1120,

## Description

### Field of invention

The present invention is directed to the use of an oligonucleotide for the preparation of a pharmaceutical composition suitable for intravenous administration for the prevention and/or treatment of tumors that are modulated by TGF-beta 2.

### Introduction

Pancreatic carcinoma is one of the most aggressive human tumors. It is almost uniformly fatal and one of the leading causes of cancer-related death in the Western world. It is more common in males than in females. In most countries, the incidence ranges from 8 to 12 cases per 100.000, with a marked increase in Japan in the last few years. The lack of clinical symptoms usually leads to a diagnosis at a late stage of the disease. At the time of diagnosis, 85 % of the patients have a locally advanced, non-resectable (stage II or stage III) or even metastatic (stage IV) carcinoma, with extra-pancreatic spread to distant organs such as liver or lung. Most patients succumb to the tumor's propensity to metastasize and its instrinsic resistance to cytotoxic agents and radiotherapy (Van Cutsem et al., 2007). Less than 3 % of the patients are still alive 5 years after diagnosis despite receiving every available therapy. Pancreatic carcinoma is accompanied by severe clinical symptoms such as cachexia, pain, obstructive jaundice, and wasting, requiring intensive supportive care.

The available treatment options are scarce. Since pancreatic carcinomas are not very sensitive to chemotherapy, therapeutic agents such as 5-FU (5-fluorouracil), gemcitabine and oxaliplatin may alleviate symptoms, but the median survival is only 6 months. Gemcitabine has been widely accepted as the gold standard chemotherapy treatment, showing a median survival of about 6 months and a 1-year survival rate of about 20 % (Burris et al., 1997). The tyrosine kinase inhibitor erlotinib, which targets epidermal-growth-factor-receptor (EGFR), was approved recently for first-line treatment of locally advanced, unresectable or metastatic pancreatic cancer in combination with gemcitabine monotherapy (Moore et al., 2007).

Another type of cancer is the malignant melanoma, which is formed by melanocytes and occurs primarily in the skin, metastasizing rapidly throughout the body. Ultraviolet damage, immunosuppression, and congenital nevi are the predominant risk factors. The occurrence of this deadliest of all skin cancers has dramatically increased over the last few years, with the highest incidence found in Australia and New Zealand. Median survival for advanced stages (distant metastases) is 7 to 8 months, depending on the organ sites affected, and the 5-year survival rate is less than 5 % (Young et al., 2006). An increased level of TGF-beta is expressed in all metastatic melanomas and in 94 % of deeply invasive primary melanomas (Reed et al., 1994).

In principal, all melanoma lesions, including lymph nodes, are removed surgically. Radiotherapy provides symptomatic relief. When metastases are present, (adjuvant) chemotherapy such as nitrosurea or dacarbazine is the first choice, although rarely with curative potential and no improvement of survival. The response rate is with 10 to 20 % rather modest and short-lived, with a response duration of about 3 months. The addition of high-dose interferon-alpha (IFN-alpha) or PEGylated IFN-alpha as immunotherapy enhances response rates, but does not influence the overall survival and involves substantial side effects. Similar findings appear to treatment with interleukin-2 (IL-2) with an even higher toxicity.

Colorectal carcinoma is a leading cause of morbidity and mortality. 5-year survival rates for stage III and IV are 67 % and 10%, respectively (SEER 2004). The highest incidence rates of colorectal cancer are found in North America, Australia/New Zealand, Western Europe, and Japan with 30.1 to 49.3 per 100,000 for men and 20.1 to 36.0 for women.

Surgery is the primary treatment for colorectal carcinoma, and adjuvant chemo-, immuno-, or radiotherapy can be added, with the FOLFOX-regimen being the first line of care. The advent of agents such as capecitabine, irinotecan, and oxaliplatin as well as targeted agents such as cetuximab, erlotinib, and bevacizumab brought prolongation of survival to the treatment of colorectal carcinoma patients. Patients with metastatic disease, treated with such novel targeted therapies, can expect a median survival approaching 2 years (Kallinowski 2005), although with some severe side effects such as diarrhea, nausea, hemorrhages and bleeding, rash, allergic reactions, and heart problems with concomitant additional management cost. Despite this progress, 5-year survival rates are still very low for stage IV patients with 10 % (SEER Program 2004), and the medical need for better treatment options is high. Multiple ongoing clinical trials are testing several combinations of chemotherapy and targeted therapies that mainly target EGFR and VEGF, with the aim to optimize current treatment. Other agents include those that inhibit the activity of the non-receptor tyrosine kinases, whereby both approaches are not specific enough on their own.

Hence, there is an urgent need for the improvement of tumor therapy, which may be reached with new compounds, combinations of known compounds, or a variation of the dosage of compounds. An overall feature of pharmaceutical substances is their increasing efficacy accompanied by an increase in amounts being administered. Particular substances used in tumor therapy show a strong correlation between the total amount administered and the inhibition of tumor growth.

Additionally, cellular uptake is a limiting factor for the efficacy of *"in vivo"* administration of oligonucleotides, where conventionally high concentrations and high amounts of oligonucleotides are administered to inhibit the production of the respective target protein.

Nevertheless, the clinical success of these substances is limited, since an increase in the concentration and the total amount of these substances, which can be administered to a mammal suffering from tumor metastases, nerval disease and immunosuppression, is correlated with a strong increase in severe side effects and toxicity.

EP 1 008 649 and EP 0 695 354 teach that oligonucleotides hybridizing with the mRNA of TGF-beta 1 and/or TGF-beta 2 can be used for manufacturing pharmaceutical compositions. EP 1 089 764 further teaches that inhibitors of substances negatively affecting the immune system in combination with oligonucleotides hybridizing with the mRNA of TGF-beta, VEGF, interleukin 10, and prostaglandin E2 and their respective receptors can be used for manufacturing a pharmaceutical composition as well. But these patents offer a wide range of concentrations, in which the oligonucleotides can be administered. WO 2006/117400 A2 specifies dosages of oligonucleotides, which are used for the preparation of a pharmaceutical composition for preventing and/or treating cancer, and wherein the oligonucleotide is suitable for local administration in low concentrations.

Similar to its function in high-grade glioma, TGF-beta (-1, -2, and/or -3) appears to play a central role in malignancy and progression of pancreatic carcinoma (Friess et al., 1993; von Berstorff, et al., 2001), malignant melanoma (Reed et al., 1994), and colorectal carcinoma, respectively. Given the limited progress, in particular clinical benefit, achieved in the treatment of pancreatic carcinoma, malignant melanoma, and colorectal carcinoma in recent years, the need for more effective therapies in terms of prolonged survival is obvious.

A solution to this problem is provided by the invention described in the following, which is directed to the use of an oligonucleotide comprising 8 to 30 nucleotide building blocks, which hybridizes with mRNA of TGF-beta 2 for use in preventing and/or treating a tumor, wherein the antisense oligonucleotide is suitable to be administered intravenously, intraarterielly, intratumorally, or subcutaneously.

### Summary of the invention

A pharmaceutical composition containing at least one oligonucleotide, which is suitable to be administered intraarterielly, intratumorally, intravenously or subcutaneously, is effective in the treatment of tumors and metastases, respectively, in particular of pancreatic carcinoma, malignant melanoma, or colorectal carcinoma, leading to a prolongation of survival compared to common pharmaceuticals.

The at least one oligonucleotide used for manufacturing a pharmaceutical preparation of this invention has a length of about 8 to about 30 nucleotide building blocks, is administered for the prevention and/or treatment of diseases that are modulated by TGF-beta 1, TGF-beta 2, TGF-beta 3, VEGF, interleukin-10, c-jun, c-fos, MIA, and/or prostaglandin E2.

In particular, the antisense oligonucleotide, which hybridizes with mRNA of TGF-beta 2, and which is used for the preparation of a pharmaceutical composition for preventing and/or treating a tumor, is suitable to be administered intravenously in a dose of 40 mg/m²/d to 190 mg/m²/d.

Surprisingly, already a low dose and a low total amount, respectively, of the pharmaceutical composition and the antisense oligonucleotide, respectively, for example a total amount of 400 to 800 mg/m²/treatment cycle, is more efficient than higher doses or total amounts of the pharmaceutical composition and the antisense oligonucleotide, respectively.

Each dose is suitable to be administered for one or more days forming cyclces of administration of different duration, for example 1 day to 12 weeks. Administration free intervals follow the administration cycles, wherein these intervals are always of the same time period or vary between the administration cycles. Preferably, the pharmaceutical composition is suitable to be administered in a cylce of 7 days followed by a 7-day treatment free period, or alternatively in a cycle of 4 days, followed by a 10-day treatment free period.

Unexpectedly, already a low dose of 80 mg/m²/d in a 7 d treatment cycle as well as a low dose of 140 mg/m²/d or 190 mg/m²/d in a 4 d treatment cycle showed high efficiency in the prolongation of survival of tumor patients.

Further advantages of the invention are enhanced affinity for nucleic acid target, enhanced cellular uptake, enhanced safety, less side effects, and increased stability in the presence of nucleases.

### Figures

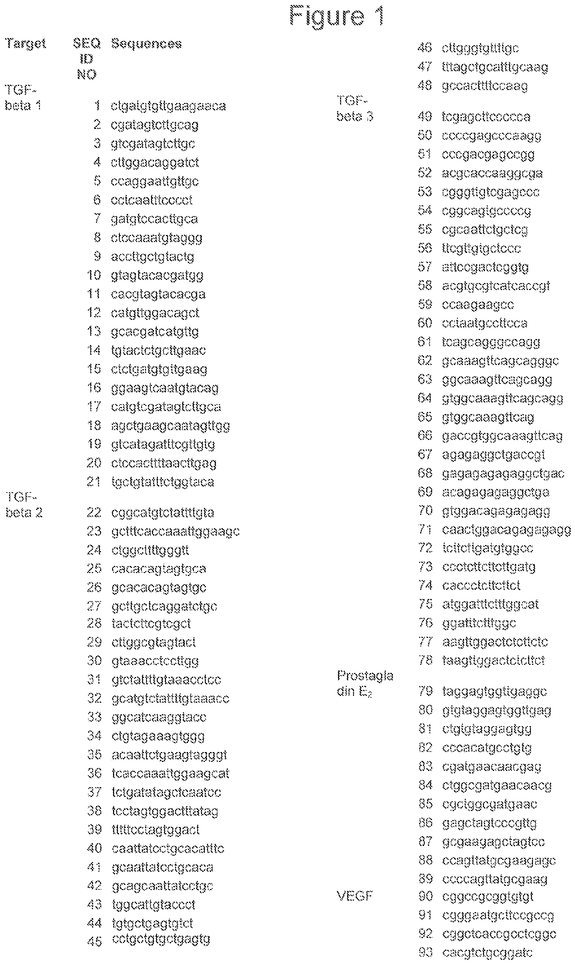

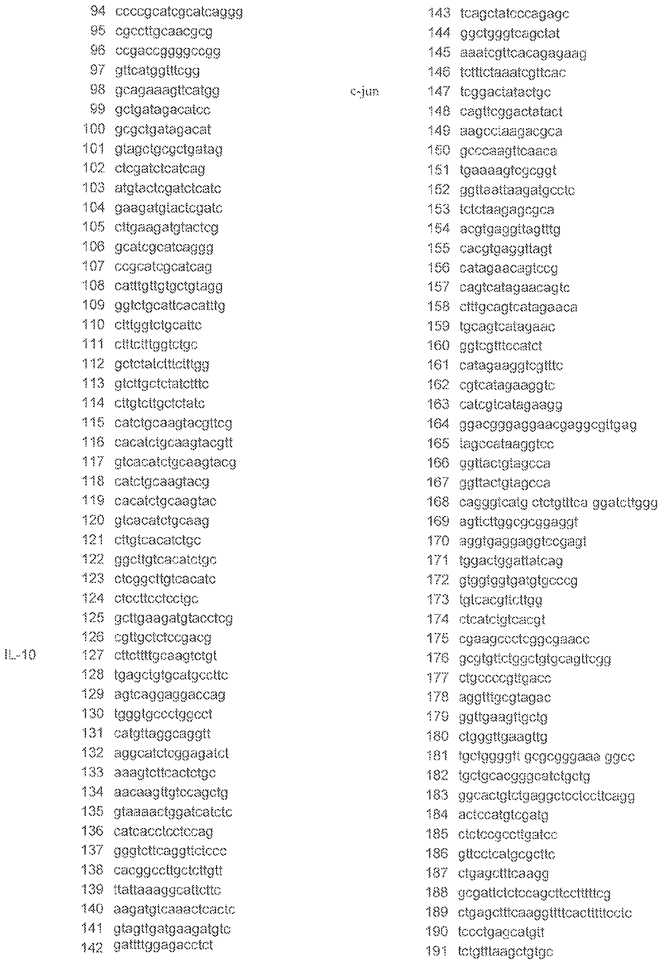

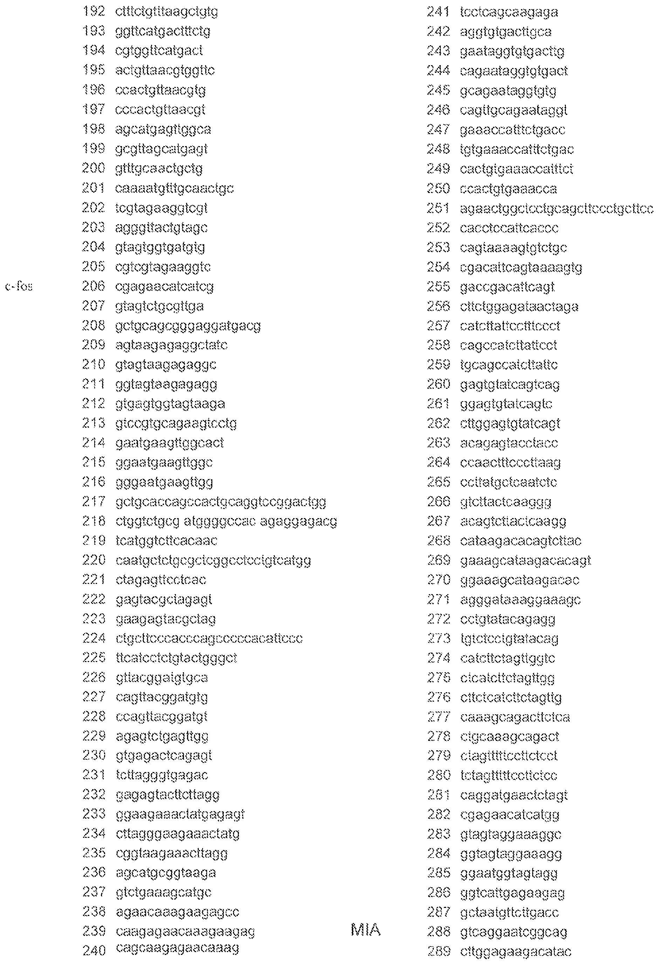

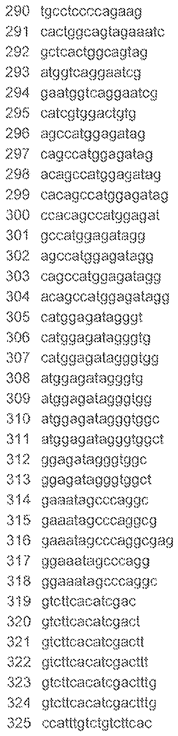

**Figure 1** presents sequences of oligonucleotides hybridizing with TGF-beta 2 (SEQ ID NO. 22 to 48) wherein oligonucleotides hybridizing with TGF-beta 2 belong to the present invention. TGF-beta 3 (SEQ ID NO. 49 to 77), prostaglandin E2 (SEQ ID NO. 78 to 89), VEGF (SEQ ID NO. 90 to 126), interleukin-10 (SEQ ID NO. 127 to 146), c-jun (SEQ ID NO. 147 to 205), c-fos (SEQ ID NO. 206 to 287), and/or MIA (SEQ ID NO. 288 to 325).

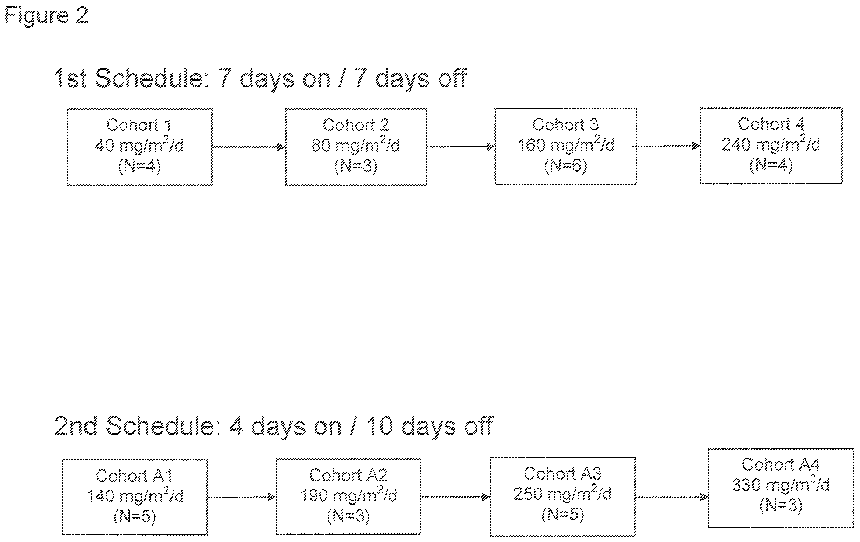

**Figure 2** shows a first treatment schedule comprising four cohorts of patients, wherein a pharmaceutical composition comprising an antisense oligonucleotide of SEQ ID NO. 22 is administered in a dose of 40 mg/m²/d, 80 mg/m²/d, 160 mg/m²/d or 240 mg/m²/d for 7 days (one cycle) followed by a 7-day treatment free period, and a second treatment schedule comprising the administration of a pharmaceutical composition comprising an antisense oligonucleotide of SEQ ID NO. 22 in a dose of 140 mg/m²/d, 190 mg/m²/d, 250 mg/m²/d, or 330 mg/m²/d for 4 days (one cycle) followed by a 10-days treatment free period.

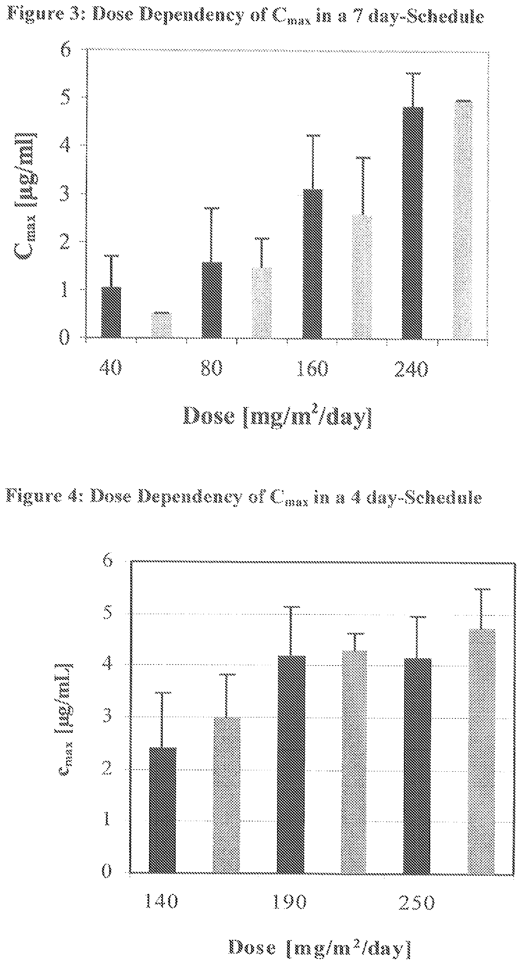

**Figure 3** presents the dose dependency of Cmax in plasma of patients, who had received an oligonucleotide in two cycles of the same dose (mg/m²) for 7 days. The patients were not treated for 7 days inbetween these treatment cycles. Different patients were treated with different doses of the antisense oligonucleotide. Black columns show the result of the first treatment cycle, grey columns show the results of the second treatment cycle.
**Figure 4** shows the dose dependency of Cmax in plasma of patients, who had received an oligonucleotide in two cycles of the same dose (mg/m²) for 4 days. The patients were not treated for 10 days inbetween these treatment cycles. Different patients were treated with different doses of the antisense oligonucleotide. Black columns show the result of the first treatment cycle, grey columns show the results of the second treatment cycle.

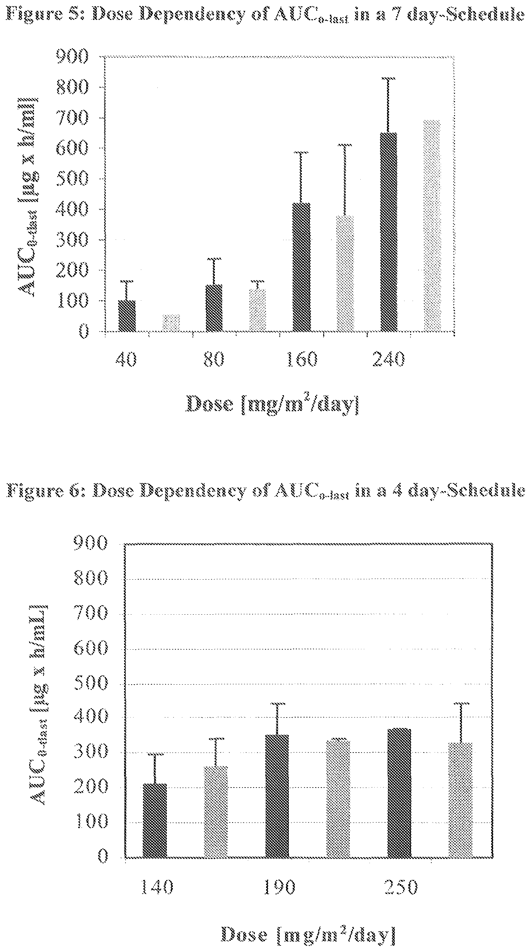

**Figure 5** demonstrates the dose dependency of the AUC in plasma of patients, who had received an oligonucleotide in two cycles of the same dose (mg/m²) for 7 days. The patients were not treated for 7 days inbetween these treatment cycles. Different patients were treated with different doses of the antisense oligonucleotide. Black columns show the result of the first treatment cycle, grey columns show the results of the second treatment cycle.
**Figure 6** presents the dose dependency of the AUC in plasma of patients, who had received an oligonucleotide in two cycles of the same dose (mg/m²) for 4 days. The patients were not treated for 10 days inbetween these treatment cycles. Different patients were treated with different doses of the antisense oligonucleotide. Black columns show the result of the first treatment cycle, grey columns show the results of the second treatment cycle.

### Detailed Description of the Invention

The expression *about* in the context of this invention comprises deviations from the absolute value given in the text from 0 to 100 %, more preferred from 1 to 80 %, even more preferred from 2 to 60 %, more preferred from 3 to 40 %, more preferred from 4 to 20 %, and even more preferred from 5 to 10 % of the respective value.

Adverse event in the context of this invention is understood as any untoward medical occurrence in a patient or clinical investigation subject after the administration of a pharmaceutical preparation that does not necessarily have a causal relationship with the treatment of the respective pharmaceutical preparation. An adverse event can therefore be any unfavourable and unintended sign (including an abnormal laboratory finding), symptom, or disease e.g. due to disease progression. Another expression for adverse event is symptom.

Pancreatic or colorectal tumor, used synonymously with tumor of the pancreas or the colorectum, or malignant melanoma according to this invention is any tumor of the pancreas, colorectum or skin including metastases, metastases derived from other parts of the pancreas, colorectum or skin or derived from any other tumor in the body.

In the present invention the expression tumor comprises any benign or malignant tumor of a human or animal body.

Hybridizing in the context of this invention implies, that two nucleotide chains at least partly form a double strand by hydrogen bonding. The double strand develops completely when the two nucleotide chains have exact antisense sequences. But even if any nucleotide of an oligonucleotide, also referred to as nucleotide building block, is substituted by another nucleotide, the respective nucleotide is modified or even when a spacer is in the place of the respective oligonucleotide, the oligonucleotide can still hybridize with the target molecule, generally the mRNA of a protein, and with this inhibit the production of said protein. Metastasis in the context of this invention means that at least one cell separates or dissociates from a tumor tissue and moves via e.g. the lymphatic system, the blood vessels, and/or invading the surrounding tissue to another part of the body of a mammal, preferably a human, where it settles down and forms new tumor tissue.

The term oligonucleotide of the invention encompasses any pharmaceutically acceptable salt, ester, or any other compound that upon administration to a mammal is capable of providing the biologically active metabolite or residue thereof. This is accomplished by specific hybridization of the compounds with one or more nucleic acids encoding TGF-beta 2,. The term oligonucleotide comprises for example an antisense oligonucleotide, siRNA, miRNA, or an aptamer.

The terms nucleic acids and oligonucleotides are used synonymously in the context of this invention.

In some embodiments, the nucleic acids are not antisense nucleic acids, meaning that they do not function by binding partly or completely to complementary DNA or RNA species, in particular genomic DNA or RNA species within a cell and thereby inhibiting the function of said DNA or RNA species, in particular genomic DNA or RNA species.

In one embodiment the oligonucleotides of this invention also comprise the oligonucleotides as described in the patents EP 0 695 354 and EP 1 008 649 as well as those of the international patent applications published under No. WO 01/68146, WO 98/33904and WO 99/63975.

Oligonucleotides hybridizing with TGF-beta 2 in one preferred embodiment include at least one sequence set forth as SEQ ID NOs: 1 to 78.

Oligonucleotides or nucleic acids include oligonucleotides having non-naturally occurring portions with similar function. Naturally occurring nucleotides as well as non-naturally occurring nucleotides, modifications and spacers are also referred to as nucleotide building block. The most common nucleotide building block is a nucleotide. Modified or substituted nucleotides as well as spacers are also comprised by the expression nucleotide building block.

These modified or substituted oligonucleotides are often preferred over native forms because of desirable properties such as enhanced cellular uptake, enhanced affinity for nucleic acid target (e.g. protein), altered intracellular localization and increased stability in the presence of nucleases. Modifications of the oligonucleotides as used herein comprise any chemical modification of the sugar, the base moiety and/or the internucleotide linkage.

In one embodiment, the ring structure of the ribose group of the nucleotides in the modified oligonucleotide or polynucleotide has oxygen in the ring structure substituted with N-H, N-R (with R being an alkyl, more preferred alkyl with 1 to 20 carbons, very preferred alkyls are methyl, ethyl, propyl, isopropyl, butyl, isobutyl, or R is an aryl substituent), S and/or methylene.

Pharmaceutical preparation in the context of this invention comprises an oligonucleotide of this invention within a pharmaceutically acceptable carrier.

In one embodiment, nucleic acids or oligonucleotides with a covalently modified base and/or sugar include for example nucleic acids having backbone sugars, which are covalently attached to low molecular weight organic groups other than a hydroxyl group at the 3' and/or 2' position or a phosphate group at the 5' position. Thus, modified nucleic acids may additionally include at least one 2'-O-substituted ribose group. For purposes of this invention, the term "2'-substituted" means substitution of the 2'-OH of the ribose molecule. The O may be substituted with N, S and the substituent can further comprise an alkyl with 1 to 20 carbons, e.g. O-alkyl, S-alkyl, NH-alkyl, N-dialkyl, O-aryl, S-aryl, NH-aryl, O-aralkyl, S-aralkyl, and NH-aralkyl. Preferred embodiments of 2'-O-alkyl-groups are methoxy-, ethoxy-, propyloxy-, isopropyloxy-, and methoxy-ethoxy. Further modifications of nucleotide building blocks are given by patents US 6,143,881, US 5,591,721, US 5,652,355, US 5,962,425, US 5,969,116 and US 5,914,396. Another preferred embodiment is a nucleotide building block, comprising at least one deoxyribose with an alkyl-group bound to the 2'-carbon. The alkyl may have about 1 to about 30 carbons, 1 to about 20 carbons, 1 to about 10 carbons, or 1 to about 5 carbons. Preferred alkyl-groups are ethyl-, propyl-, isopropyl-, butyl-group, highly preferred is the methyl-group.

In yet another embodiment, modified nucleic acids include sugars such as arabinose instead of ribose. Thus the nucleic acids may be heterogeneous in backbone composition, thereby containing any possible combination of polymer units linked together such as peptide-nucleic acids (which have amino acid backbone linked to nucleic acid bases). In some embodiments the nucleic acids are homogeneous in backbone composition.

The substituted purines and pyrimidines of the nucleic acids include standard purines and pyrimidines such as cytosine as well as base analogs such as substituted bases (Wagner et al. 1993). Purines and pyrimidines include, but are not limited to adenine, cytosine, guanine, thymine, inosin, 5-methylcytosine, 2-aminopurine, 2-amino-6-chloropurine, 2,6-diaminopurine, hypoxanthine, and other naturally and non-naturally occurring nucleobases, substituted and unsubstituted aromatic moieties.

The single nucleotides in each oligonucleotide may contain the same modifications, may contain combinations of these modifications, or may combine these modifications with phosphodiester linkages. Methods of rendering oligonucleotide nuclease resistant include, but are not limited to, covalently modifying the purine or pyrimidine bases. For example, bases may be methylated, hydroxymethylated, or otherwise substituted (e.g., glycosylated) such that the oligonucleotides or polynucleotides are rendered substantially acid and nuclease resistant.

In a preferred embodiment, at least one end of the oligonucleotide is a biotin, biotin analog, avidin, or avidin analog. These molecules have the ability to block the degradation of the protected oligonucleotide and provide means for high affinity attachment of the modified nucleic acids to the solid support. Avidin and biotin derivatives, which can be used to prepare the reagents of this invention, include streptavidin, succinylated avidin, monomeric avidin, biocytin (biotin-epsilon-N-lysine), biocytin hydrazide, amine or sulfhydryl derivatives of 2-iminobiotin and biotinyl-epsilon-aminocaproic acid hydrazide. Additional biotin derivatives, such as biotin-N-hydroxysuccinimide ester, biotinyl-epsilon-aminocaproic acid-N-hydroxysuccinimide ester, sulfosuccinimidyl 6-(biotin amido)hexanoate, N-hydroxysuccinimideiminobiotin, biotinbromoacetylhydrazide, p-diazobenzoyl biocytin and 3-(N-maleimidopropionyl)-biocytin, can also be used as end-blocking groups on the oligonucleotides of the present invention.

In a further preferred embodiment, the oligonucleotide is conjugated with a polymer, in particular PEG, at the 3'- and/or 5'-end of the oligonucleotide and/or at any internal nucleotide of the oligonucleotide as described in WO 2008/077956 A2.

In an alternative embodiment the oligonucleotide is an aptamer or a gapmer interacting with a DNA or RNA sequence.

In yet another embodiment, the base units are maintained for hybridization with an appropriate nucleic acid target compound. One such oligomeric compound, an oligonucleotide mimetic that has been shown to have excellent hybridization properties, is referred to as a peptide nucleic acid (PNA). In PNA compounds, the sugar-backbone of an oligonucleotide is replaced with an amide containing backbone, in particular an aminoethylglycine backbone. The nucleobases are bound directly or indirectly to aza nitrogen atoms of the amide portion of the backbone. Representative United States patents that teach the preparation of PNA compounds include, but are not limited to, US 5,539,082, US 5,714,331, and US 5,719,262. Further teaching of PNA compounds can be found in Nielsen et al. 1991.

Further backbone modified oligonucleotide includes, for example, phosphorothioates, chiral phosphorothioates, phosphorodithioates, phosphorotriesters, aminoalkyl-phosphorotriesters, methyl- and other alkyl-phosphonates including 3'-alkylene phosphonates and chiral phosphonates, phosphinates, phosphoramidates, including 3'-aminophosphoramidate and aminoalkylphosphoramidates, thiono-phosphor-amidates, thionoalkylphosphonates, thionoalkylphosphotriesters, and boranophosphates having normal 3'-5'linkages, 2'-5'linked analogs of these, and those having inverted polarity, wherein the adjacent pairs of nucleoside units are linked 3'-5' to 5'-3' or 2'-5' to 5'-2'. Various salts, mixed salts, and free acid forms are also included. One preferred embodiment is the sodium salt of the nucleic acid.

In some embodiments at least one nucleotide of an oligonucleotide is modified as described in one of the modifications above. The modification can either cover the oligonucleotide continuously or irregularly.

In yet another embodiment, at least two modifications as described above are combined within one oligonucleotide.

In another embodiment, 1 to about 12 or 1 to about 8 or 1 to about 4 or 1 to about 2 oligonucleotides and/or nucleotide linkages at the 3' and/or 5'end of the oligonucleotide are modified as described above.

In one embodiment the oligonucleotide of this invention hybridizes with its target TGF-beta 2. The antisense structure of the mRNA of said target is described in PCT/EP2004/053604.

Chain elongation means that oligonucleotides of the sequence listing and other oligonucleotides, that have additional nucleotides of the sequence of the respective antisense structure of the mRNA of said targets, are still within the scope of this invention. The additional nucleotides in one embodiment are according to the coding region of the mRNA, in yet another embodiment, the additional nucleotides are also from the non-coding part of the mRNA, including introns and exons. The additional nucleotides comprise about 1 to about 10,000 nucleotides, about 1 to about 5,000 nucleotides, about 1 to about 3,000 nucleotides, about 1 to about 1,000 nucleotides, about 1 to about 500 nucleotides, about 1 to about 100 nucleotides, about 1 to about 50 nucleotides, about 1 to about 25 nucleotides, about 1 to about 10 nucleotides, about 1 to about 5 nucleotides or about 1 to about 2 nucleotides bound to at least one of the 3' and/or 5' end, in another embodiment, to at least one of the 2' or 5'end. In yet another embodiment, some nucleotide building blocks of these oligonucleotides or polynucleotides may be modified or substituted by spacers and/or modifications as described herein.

Pharmaceutically acceptable salts refer to physiologically and pharmaceutically acceptable salts of the compounds used in the invention, which means that the salts retain the biological activities of the parent compounds without undesired toxicological effects.

In one embodiment the oligonucleotide or its active derivative is a single stranded oligonucleotide, in yet another embodiment the oligonucleotide is double stranded, which means that the oligonucleotide is hybridized completely or partly with a second oligonucleotide that has about 50 % to about 100 % of the exact antisense structure of said oligonucleotide. This may result in a double strand, in which both oligonucleotides hybridize with less bond strength or in double strand with overlapping ends. The two oligonucleotides might have the same length, in other words they have the same amount of nucleotide building blocks or their length might differ, also resulting in overlapping ends on one or both ends.

The expression spacer in the context of this invention comprises any nucleotide building block, which is not obviously a derivative or a modification of a nucleotide, but connects two nucleotide building blocks in a way, that the resulting oligonucleotide still hybridizes with its target, mRNA of TGF-beta 2.

One embodiment of the invention refers to the use of an oligonucleotide, preferably an antisense oligonucleotide, comprising 8 to 30 nucleotide building blocks, which hybridizes with mRNA of TGF-beta 2 for use in preventing and/or treating a tumor, wherein the antisense oligonucleotide is administered intravenously in a dose of 80 mg/m²/d, 140 mg/m²/d or 190 mg/m²/d, preferably for a prolongation of survival of at least 0.5 to 40 months, of at least 1 to 40 months, or of at least 3 to 40 months.

In other embodiments of the invention, the oligonucleotide is administered preferably in a dose of 40 mg/m²/d to 400 mg/m²/d, more preferred in a dose of 40 mg/m²/d to 250 mg/m²/d, 80 mg/m²/d to 250 mg/m²/d, 140 mg/m²/d to 250 mg/m²/d, 160 mg/m²/d to 250 mg/m²/d, 190 mg/m²/d to 250 mg/m²/d, or 40 mg/m²/d to 190 mg/m²/d, 80 mg/m²/d to 190 mg/m²/d, 160 mg/m²/d to 190 mg/m²/d, or 40 mg/m²/d to 160 mg/m²/d, 80 mg/m²/d to 160 mg/m²/d, or even more preferred in a dose of 40 mg/m²/d to 140 mg/m²/d, 80 mg/m²/d to 140 mg/m²/d. The most preferred doses of the oligonucleotide are 40 mg/m²/d, 60 mg/m²/d, 80 mg/m²/d, 100 mg/m²/d, 120 mg/m²/d, 140 mg/m²/d, 160 mg/m²/d, 180 mg/m²/d, 190 mg/m²/d, 200 mg/m²/d, 210 mg/m²/d, 220 mg/m²/d, 230 mg/m²/d, 240 mg/m²/d, 250 mg/m²/d, 300 mg/m²/d, 350 mg/m²/d, 400 mg/m²/d, 450 mg/m²/d, or 500 mg/m²/d.

In a preferred embodiment an oligonucleotide is in a dose of 40 mg/m²/d, 80 mg/m²/d, 140 mg/m²/d, 160 mg/m²/d, 190 mg/m²/d, 240 mg/m²/d, 250 mg/m²/d, 300 mg/m²/d, 330 mg/m²/d, or 350 mg/m²/d.

In another embodiment of the invention, the pharmaceutical composition and the antisense oligonucleotide in the above mentioned doses, respectively, is able to prolong survival of a patient suffering from a malignant tumor such as pancreatic carcinoma, malignant melanoma, or colorectal carcinoma for at least or about 0.5 to 60 months, 0.5 to 48 months, 0.5 to 36 months, 0.5 to 24 months, 0.5 to 12 months, 0.5 to 9 months, 0.5 to 6 months, or 1 to 60 months, 1 to 48 months, 1 to 36 months, 1 to 24 months, 1 to 12 months, 1 to 9 months, 1 to 6 months, preferably at least or about 2 to 60 months, 2 to 48 months, 2 to 36 months, 2 to 24 months, 2 to 12 months, 2 to 9 months, 2 to 6 months, more preferred at least or about 3 to 60 months, 3 to 48 months, 3 to 36 months, 3 to 24 months, 3 to 12 months, 3 to 9 months, even more preferred for at least or about 0.5, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60 months, most preferred for at least 10 years, 15 years, 20 years, 25 years, 30 years, 35 years, 40 years, 45 years, 50 years, 55 years, 60 years, 65 years, 70 years, 75 years, 80 years, 85 years, 90 years, 95 years, 100 years.

Whereas the oligonucleotides of this invention are single stranded, in some embodiments at least parts of the single-stranded nucleic acid are double-stranded. Double-stranded molecules are more stable in vivo, while single-stranded molecules have increased activity.

In one embodiment the oligonucleotide has a length between about 8 to about 30 nucleotides and is complementary to the mRNA of the target TGF-beta 2. In more preferred embodiments, the oligonucleotides of this invention have lengths of about 7 to about 25 nucleotides, of about 8 to about 30 nucleotides, even more preferred of about 12 to 18 nucleotides. Preferred embodiments of oligonucleotides usable in this invention are shown in the sequence listing under SEQ ID NOs 1 to 78, very preferred embodiments are SEQ ID NOs 22 to 48, even more preferred is SEQ ID NOs 1 or 22 or 58. In one preferred embodiment SEQ ID NO 22 is administered in the form of its sodium salt having a molecular weight of 6142.4 g, respectively 7115.3 g as hydrate, comprising about 54 molecules of crystal water per oligonucleotide.

Other oligonucleotides usable in pharmaceutical preparations according to this invention are oligonucleotides described in the sequence listing, respectively, the examples of EP 1 089 764, PCT/EP98/00497 and PCT/EP2005/002101.

In other embodiments the at least one oligonucleotide for the manufacturing of a pharmaceutical preparation comprises at least one phosphorothioate linkage between two nucleotide building blocks. The phosphorothioate linkage may cover 0 % - 5%, 5 % to 10%, 10 % to 15 %, 15 % to 20 %, 20 % to 25 %, 25 % to 30 %, 30 % to 35%, 35 % to 40 %, 40 % to 45 %, 45 % to 50 %, 50 % to 55%, 55 % to 60 %, 60 % to 65 %, 65 % to 70 %, 70 % to 75 %, 75 % to 80 %, 80 % to 85 %, 85 % to 90 %, 90 % to 95 % or 95 % to 100 % of the linkages. The phosphorothiate linkages may be scattered regularly or irregularly over the oligonucleotide. In some embodiments the phosphorothioate is accumulated at either one or both of the 3' and/or 5' ends of the oligonucleotide.

Another preferred embodiment is an oligonucleotide where all linkages between the nucleotides are phosphorothioates.

In one embodiment the oligonucleotide is a single strand oligonucleotide. Single strand means, that all oligonucleotide building blocks are in one line and do not hybridize with a second oligonucleotide or antisense oligonucleotide and are not bound otherwise.

In a preferred embodiment the pharmaceutical composition and the oligonucleotide, respectively, is administered intravenously, or by infusion into a tissue, a tumor or a body cavity. In more preferred embodiments the tissue is selected from the group of bile duct, bladder, bone, bone marrow, brain, breast, colon, endometrium, epithelium, gall bladder, head, head and neck, heart, intestine, joints, kidney, larynx, liver, lung, lymphatic node, lymphatic vessels, muscle, oesophagus, ovary, pancreas, prostate, rectum, urinary duct, skin and its layers, spleen, stomach, testis, thymus, thyroid, tonsil, or uterus and/or is administered to the respective tumor of this tissue.

In another embodiment the pharmaceutical preparation of oligonucleotides is administered into a body cavity, selected from the group of bile duct, bladder, colon, gall bladder, joint cavity, pleural cavity, intraperitoneal space, the rectum, the intestinal tract, the urinary duct, the urinary bladder, and the ventricular space, in particular the cerebro ventricular space.

In yet other embodiments of this invention the pharmaceutical composition comprising at least one oligonucleotide is administered for example intravenously with a flow rate of about0.01 to about 1 ml/h, 0.01 to about 10 ml/h, about 0.1 ml/h to about 1 mL/h, about 0.1 ml/h to about 10 mL/h, 1 mL/h to 10 mL/h, or 5 mL/h to 10 mL/h. Further preferred are flow rates of about 0.2 mL/min to about 9 mL/min, 0.3 mL/h to 8 mL/h, 0.4 mL/h to 7 mL/h, 0.5 mL/h to 6 mL/h, 0.6 mL/h to 5 mL/h, 0.7 mL/h to 5 mL/h, 0.8 mL/h to 5 mL/h, 0.9 mL/h to 5 mL/h, more preferred of about 0.1 mL/h, 0.2 mL/h, 0.25 mL/h, 0.3 mL/h, 0.4 mL/h, 0.5 mL/h, 0.6 mL/h, 0.7 mL/h, 0.8 mL/h, 0.9 mL/h, 1.0 mL/h, 1.1 mL/h, 1.2 mL/h, 1.3 mL/h, 1.4 mL/h, 1.5 mL/h, 1.6 mL/h, 1.7 mL/h, 1.8 mL/h, 1.9 mL/h, or 2.0 mL/h.

In one embodiment, the pharmaceutical composition is suitable to be administered with an application system such as a syringe. A preferred application system comprises a portable pump, connected with flexible tubes to a port system. The port system is connected to an infusion catheter. Application systems usable for this invention are for example described in the international patent application PCT/EP 2004/ 004211.

Preferably, the total volume per day of the pharmaceutical composition and the oligonucleotide, respectively, which is administered for example intravenously ranges from about 1 mL/d to about 500 mL/d, about 2 mL/d to about 250 mL/d, more preferred from about 5 mL/d to about 200 mL/d, even more preferred from about 7 mL/d to about 150 mL/d, even more preferred from about 10 mL/d to about 100 mL/d, even more preferred from about 15 mL/d to about 50 mL/d, and most preferred from about 20 mL/d or about 50 mL/d.

In one embodiment the pharmaceutical composition and the oligonucleotide, respectively, is suitable to be administered for example intravenously or into a tumor, a tissue or a body cavity in 1 to 15 cycles, 1 to 10 cycles, preferably in 2 to 10 cycles, 2 to 8 cycles, 2 to 6 cycles, or 2 to 4 cycles, more preferably in 1, 2, 3, 4, 5, 6, 7, 8, 9, 10, 11, 12, 13, 14, 15 cycles, which are administered in one or more days and the duration of each cycle is identical or varies.

In a preferred embodiment, the pharmaceutical composition and the antisense oligonucleotide, respectively, were administered in at least or about 24 h, 2d, 3d, 4d, 5d, 6d, 7d, 8d, 9d, 10d, 11d, 12d, 13d, 14d, 15d.
More preferred, the pharmaceutical composition and the antisense oligonucleotide, respectively, were administered for example intravenously in at least or about 1d, 2d, 3d, 4d, 5d, 6d, 7d, 8d, 9d, 10d, 11d, 12d, 13d, 14d, 15d, as one treatment cycle, wherein the pharmaceutical composition and the antisense oligonucleotide, respectively, is suitable to be administered on consecutive days, every second day, every third day, every forth day, every fifth day, every sixth day, every seventh day, in consecutive weeks, every second week, every third week, every forth week, every fifth week, every sixth week, every seventh week, every eighth week, every nineth week, every tenth week, every eleventh week, or every twelfth week.

In preferred embodiments, the time interval between one treatment cycle and another cycle is at least or about 24h, 2d, 3d, 4d, 5d, 6d, 7d, 8d, 9d, 10d, 11d, 12d, 13d, 14d, 15d, wherein the time intervals between the treatment cycles are either identical or vary. During these time intervals between the administration cycles, no pharmaceutical composition and antisense oligonucleotide, respectively, is administered. Alternatively, during the time intervals between the administration cycles another compound such as a chemotherapeutic, for example 5-fluorouracil, BCNU, or a vinca alkaloid, is administered.

In one embodiment the bioavailability of the administered oligonucleotide in a cell or in plasma of a subject unexpectedly increases with a decrease in the duration of the treatment cycle, if the same total amount of the oligonucleotide is administered. The concentration of the oligonucleotide in the cell or the plasma of a patient increases with increasing dose of the administered oligonucleotide independent of the duration of the treatment cycle (Fig. 3 and 4). Cₘₐₓ is defined as the maximal concentration of a substance in a medium, and indicates in Fig. 3 and 4 the maximal concentration of an antisense oligonucleotide, in particular a TGF-beta 2 antisense oligonucleotide in the blood plasma of a subject.

In this preferred embodiment, an increasing dose of the oligonucleotide results in an increase of the bioavailability of this oligonucleotide in a cell or plasma of a subject, wherein the same total amount of an oligonucleotide results in a higher bioavailability, if the duration of the treatment cycle is reduced (Fig. 5 and 6). Bioavailability is a measurement of the extent of a therapeutically active drug, for example an antisense oligonucleotide that reaches the systemic circulation and is available at the site of action. Bioavailability is analysed by the AUC (area under the curve), which is defined as the area under the curve of the plasma concentration in a determined time period, e.g., in Example 6 four or seven days.

In a most preferred embodiment, the antisense oligonucleotide, for example dissolved in a physiological (= isotonic) solution, is administered in at least or about 1, 2, 3, 4, 5, 6, 7, 8, 9, 10, 11, 12, 13, 14 or 15 cycles, in 1 or 2 to 10 cycles, 1 or 2 to 8 cycles, 1 or 2 to 6 cycles, 1 or 2 to 4 cycles, 1 or 2 cycles, wherein one cycle consists of 4d and the time interval between the cycles is 10d, or wherein one cycle consists of 4d and the time interval between the cycles is 7d, or wherein one cycle consists of 10d and the time interval between the cycles is 4d, or wherein one cycle consists of 7d and the time interval between the cycles is 7d, or wherein one cycle consists of 7d and the time interval between the cycles is 10d, or wherein one cycle consists of 10d and the time interval between the cycles is 7d wherein the tumor is preferably pancreatic carcinoma, malignant melanoma, or colorectal carcinoma.

The pharmaceutical composition and the antisense oligonucleotide, respectively, is administered in a total amount of at least or about 400 to 800 mg / m² /cycle, more preferred of at least or about 400, 500, 600, 700, 800 mg / m² / cycle, and most preferred of at least or about 510, 520, 530, 540, 550, 560, 570, 580, or 590 mg / m² / cycle.

Most surprising of the present invention is that not the highest dose and amount, respectively, of the antisense oligonucleotide shows the best effects or that the treatment effects increase with an increased dose of the antisense oligonucleotide, but that lower amounts of for example 560 mg / m² / cycle present the best effects in the treatment of tumors (see examples of the invention).

The pharmaceutical composition and the antisense oligonucleotide, respectively, is used as a first line treatment, i.e, without any tumor therapy in advance, as a second line treatment, i.e., with one tumor therapy in advance, as a third line treatment, i.e., with two tumor therapies in advance, or as a fourth line treatment, i.e., with three tumor therapies in advance, or as at least a fifth line treatment with at least four tumor therapies in advance, wherein the preceding tumor therapy is for example a chemotherapy or a radiotherapy.

In yet another embodiment the pharmaceutical composition comprises at least one oligonucleotide and in addition at least one further adjuvant treatment. For more details about adjuvants, also referred to as immunostimulants, see EP 1 089 764. In a preferred embodiment the adjuvant is an oligonucleotide comprising a CpG-motive. For further details about CpG motives see Krieg 2002.

Preferred embodiments of the pharmaceutical composition of this invention are used in mammals, where the preferred embodiment of mammal is a human. Besides being useful in human treatment, the present invention is also useful for other subjects including veterinary animals, exotic animals and farm animals, including mammals, rodents, and the like. Mammals include humans, horses, dogs, pigs, cats, or primates (for example, a monkey, a chimpanzee, or a lemur). Rodents include rats, mice, squirrels, or guinea pigs.

Pharmaceutical compositions include fluid pharmaceutical compositions containing the oligonucleotide in a concentration of at least or about 1 mM to about 25 M, 1 mM to about 20 M, 1 mM to about 10 M, 1 mM to about 5 M, 1 mM to about 1 M. In another preferred embodiment the pharmaceutical composition contains the oligonucleotide in a concentration of at least or about 10 mM, 20 mM, 30 mM, 40 mM, 50 mM, 60 mM, 70 mM, 80 mM, 90 mM or 100 mM to about 150 mM, 200 mM, 250 mM, 300 mM, 350 mM, 400 mM, 450 mM, 500 mM, 550 mM, 600 mM, 650 mM, 700 mM, 750 mM, 800 mM, 850 mM, 900 mM, 950 mM, 1 M, 2, M, 3 M, 4 M, 5 M, 6 M, 7 M, 8 M, 9 M, 10 M, 11 M, 12 M, 13 M, 14 M, 15 M, 16 M, 17 M, 18 M, 19 M, or 20 M.

The pharmaceutical composition may include sterile aqueous solutions that may also contain buffers, diluents and other suitable additives, such as, but not limited to, penetration enhancers, acceptable carriers or excipients. The term pharmaceutical composition implies that the liquids or substances of this composition are pure and/or combined with pharmaceutically acceptable carriers.

The term pharmaceutically acceptable carrier means one or more compatible liquid fillers, diluents or encapsulating substances that are suitable for administration to a human or other mammal. The term "carrier" denotes an organic or inorganic ingredient, natural or synthetic, with which the active ingredient is combined to facilitate the application. The components of the pharmaceutical composition are capable of being combined with the oligonucleotides of the present invention without negatively influencing the desired pharmaceutical efficacy. Such carriers enable the oligonucleotides of the invention to be formulated as liquids, gels, syrups, slurries, suspensions, or emulsions.

As described above the pharmaceutical compositions may also include microcapsules, nanocapsules, micro- and nanospheres, and suspensions, oligonucleotides coated onto microscopic gold particles or preparations with protracted release of oligonucleotides, in whose preparation excipients and additives and/or auxiliaries such as disintegrants, binders, coating agents, swelling agents, lubricants, or solubilizers are customarily used. For the infusion into the stomach, intestine, colon, or rectum for example an enema is useful. For a brief review of present methods of drug delivery, see Langer (1990).

In yet another embodiment, a suspension of the compounds is prepared as appropriate oily infusion. Suitable lipophilic solvents or vehicles include fatty oils such as sesame oil, or synthetic fatty acid esters, such as ethyl oleate or triglycerides, or liposomes. Aqueous infusion suspensions comprise substances, which increase the viscosity of the suspension, such as sodium carboxymethyl cellulose, sorbitol, or dextran. Optionally, the suspension may also contain suitable stabilizers or agents, which increase the solubility of the compounds to allow for the preparation of highly concentrated solutions.

In yet another embodiment, the oligonucleotides may be in powder form for reconstitution with a suitable vehicle, e.g., sterile pyrogen-free water, before use. In other embodiments the oligonucleotide is reconstituted with an isotonic solution. Isotonic solution is any aqueous solution with an osmotic pressure as high as the osmotic pressure of the body. Commonly used are solutions of salts, sugars and so on. In preferred embodiments the isotonic solution is a 0.9 % sodium chloride solution or a Ringer-lactate solution (e. g. DAB7).

In yet another embodiment, the active compounds, e.g., the oligonucleotide, may be in form of a concentrate for dilution with a suitable vehicle, e.g., sterile pyrogen-free water, before use. In yet another embodiment, the compounds are formulated in rectal or vaginal compositions such as suppositories or retention enemas or tablets, e.g., containing conventional suppository bases such as cocoa butter or other glycerides.

In yet another embodiment, the compounds are formulated as a depot preparation. In one embodiment such long acting preparations are formulated with suitable polymeric or hydrophobic materials (for example as an emulsion in an acceptable oil) or ion exchange resins, or as sparingly slow soluble derivatives, for example as a sparingly slow soluble salt.

In other embodiments, delivery systems include time-release, delayed release, or sustained release delivery systems. Such systems can avoid repeated administrations of the compounds, thereby providing increasing convenience to the subject and the physician. Many types of release delivery systems are available and known to those of ordinary skill in the art.

In one embodiment, the delivery systems include polymer based systems such as poly(lactide-glycolide), copolyoxalates, polycaprolactones, polyesteramides, polyorthoesters, polyhydroxybutyric acid, and polyanhydrides, more preferred polyanhydrides with molecular weight of more than 20,000 Da. In one preferred embodiment the polyanhydride is a polycondensation of dicarbonic acids with high molecular weight. More details see also EP 0 260 415. Microcapsules of the foregoing polymers containing drugs are described for example in U.S. Pat. No. 5,075,109.

In another embodiment, improvement of oligonucleotide uptake has been achieved with different systems of vectorization including liposomes (neutral, cationic, immunoliposome), micro- and nanoparticles, polymers, or covalent attachment to a carrier (Lefebure et al., 1995). Lipid-mediated transfection has also been used to deliver oligonucleotides (Wang and Martini, 1996). In another embodiment the delivery systems include non-polymer systems that are e.g., lipids including sterols such as cholesterol, cholesterol esters and fatty acids or neutral fats such as mono-, di- and triglycerides, hydrogel release systems, silastic systems, peptide based systems, and the like.

Specific examples include, but are not limited to: (a) erosional systems, in which an agent of the invention is contained in a form within a matrix such as those described in US 4,452,775, US 4,675,189, and US 5,736,152, and (b) diffusional systems, in which an active component permeates at a controlled rate from a polymer such as described in US 3,854,480, US 5,133,974 and US 5,407,686.

In still other embodiments, the oligonucleotides are formulated with GELFOAM®, a commercial product consisting of modified collagen fibers that degrade slowly.
In one embodiment, the pharmaceutical compositions also comprise suitable solid or gel phase carriers or excipients. Examples of such carriers or excipients include, but are not limited to calcium carbonate, calcium phosphate, various sugars, starches, cellulose derivatives, gelatin, and polymers such as polyethylene glycols.

In one embodiment, the oligodeoxynucleotides are administered neat or in the form of a pharmaceutically acceptable salt. The salts have to be pharmaceutically acceptable, but non-pharmaceutically acceptable salts may conveniently be used to prepare pharmaceutically acceptable salts thereof. Such salts include, but are not limited to, those prepared from the following acids: hydrochloric, hydrobromic, sulphuric, nitric, phosphoric, maleic, acetic, salicylic, p-toluene sulphonic, tartaric, citric, methane sulphonic, formic, malonic, succinic, naphthalene-2-sulphonic, and benzene sulphonic acid. Also, such salts can be prepared as alkaline metal or alkaline earth salts, such as sodium, potassium or calcium salts of the carboxylic acid group.

In another embodiment, the pharmaceutical composition further comprises at least one buffer and/or at least one preservative. In one embodiment suitable buffering agents include but are not limited to acetic acid and a salt (e.g., 1-2% w/v), citric acid and a salt (e.g., 1-3% w/v), boric acid and a salt (e.g., 0.5-2.5% w/v), and phosphoric acid and a salt (e.g., 0.8-2% w/v). Suitable preservatives include benzalkonium chloride (e.g., 0.003-0.03% w/v), chlorobutanol (e.g., 0.3-0.9% w/v), parabens (e.g., 0.01-0.25% w/v), and thiomersal (e.g., 0.004-0.02% w/v).

In yet another embodiment the pharmaceutical compositions also include penetration enhancers in order to enhance the alimentary delivery. Penetration enhancers may be classified as belonging to one of five broad categories, i.e., fatty acids, bile salts, chelating agents, surfactants, and non-surfactants (Lee et al., 1991; Muranishi, 1990). One or more penetration enhancers from one or more of these broad categories may be included.

Various fatty acids and their derivatives, which act as penetration enhancers, include, for example, oleic acid, lauric acid, capric acid, myristic acid, palmitic acid, stearic acid, linoleic acid, linolenic acid, dicaprate, tricaprate, recinleate, monoolein (1-monooleoyl-rac-glycerol), dilaurin, caprylic acid, araichidonic acid, glyceryl 1-monocaprate, 1-dodecylazacycloheptan-2-one, acylcarnitines, acylcholines, mono- and di-glycerides and physiologically acceptable salts thereof (i.e., oleate, laurate, caprate, myristate, palmitate, stearate, linoleate, etc.) (Lee et al., 1991; Muranishi, 1990; El-Hariri et al., 1992). Examples of some presently preferred fatty acids are sodium caprate and sodium laurate, used singly or in combination at concentrations of for example 0.5 to 5%.

The physiological roles of bile include the facilitation of dispersion and absorption of lipids and fat-soluble vitamins (Brunton, 1996). Various natural bile salts, and their synthetic derivatives, act as penetration enhancers. Thus, the term "bile salt" includes any of the naturally occurring components of bile as well as any of their synthetic derivatives. A presently preferred bile salt is chenodeoxycholic acid (CDCA) (Sigma Chemical Company, St. Louis, Mo.), used in concentrations of for example 0.5 to 2%.

Complex formulations comprising one or more penetration enhancers may be used. For example, bile salts may be used in combination with fatty acids to produce complex formulations. Preferred combinations include CDCA combined with sodium caprate or sodium laurate (e.g., 0.5 to 5%).

In one embodiment, additionally chelating agents are used that include, but are not limited to, disodium ethylenediaminetetraacetate (EDTA), citric acid, salicylates (e.g., sodium salicylate, 5-methoxysalicylate and homovanillate), N-acyl derivatives of collagen, laureth-9 and N-amino acyl derivatives of beta-diketones (enamines) (Lee et al., 1991; Muranishi, 1990; Buur et al. 1990). Chelating agents have the added advantage of also serving as DNase inhibitors.

In yet another embodiment additionally surfactants are used. Surfactants include, for example, sodium lauryl sulphate, polyoxyethylene-9-lauryl ether and polyoxyethylene-20-cetyl ether (Lee et al., 1991), and perfluorochemical emulsions such as FC-43 (Takahashi et al., 1988).

Non-surfactants include, for example, unsaturated cyclic ureas, 1-alkyl- and 1-alkenylazacyclo-alkanone derivatives (Lee et al., 1991), and non-steroidal anti-inflammatory agents such as diclofenac sodium, indomethacin, and phenylbutazone (Yamashita et al., 1987).

In one embodiment, the pharmaceutical composition of the present invention additionally contains other adjunct components conventionally found in pharmaceutical compositions, at their art-established usage levels. Thus, for example, the compositions may contain additional compatible pharmaceutically active materials such as, e.g., antipruritics, astringents, local anesthetics or anti-inflammatory agents, or may contain additional materials useful in physically formulating various dosage forms of the composition of present invention, such as dyes, flavoring agents, preservatives, antioxidants, opacifiers, thickening agents, and stabilizers. However, such materials, when added, should not unduly interfere with the biological activities of the components of the compositions of the invention.

### Examples

In these examples the results of systemic toxicity studies as well as clinical studies in patients are reported. All these studies have been performed in accordance with current Good Clinical Practice (GCP) guidelines and have been approved by local ethics committees. Patient studies furthermore have followed the current international declaration of Helsinki for human experimentation.

The following examples represent specific embodiments of the invention; however, the present invention is not limited to these examples.

### Example 1

### Effect of an antisense oligonucleotide in a 7-day treatment cycle on pancreatic carcinoma

A pharmaceutical composition comprising an antisense oligonucleotide of SEQ ID NO. 22 was administered intravenously as a 2^{nd,}, 3^{rd} or 4^{th} line treatment to a patient suffering from an advanced pancreatic carcinoma, i.e., the patients had been pretreated with one, two or three other tumor therapies. A portable pump was used to infuse the pharmaceutical composition and the antisense oligonucleotide of SEQ ID No. 22, respectively, intravenously at an infusion speed of 0.8 mL/h via a pre-implanted port access system. Alternatively, the pharmaceutical composition and the antisense oligonucleotide of SEQ ID No. 22, respectively, were administered intravenously with a syringe.

The pharmaceutical composition comprising the antisense oligonucleotide of SEQ ID NO. 22 is administered intravenously in 1 to 10 treatment cycles, wherein one cycle consists of 7 days, followed by a 7-day treatment free interval.

17 patients were treated with this treatment schedule, i.e., receiving a 7-day continuous intravenous infusion of the pharmaceutical composition comprising the antisense oligonucleotide of SEQ ID No. 22, followed by a 7-day interval free of treatment. These patients were enrolled in four successive cohorts with increasing doses:
Cohort 1: 4 patients received 40 mg/m²/d (i.e., 280 mg/m²/cycle)
Cohort 2: 3 patients received 80 mg/m²/d (i.e., 560 mg/m²/cycle)
Cohort 3: 6 patients received 160 mg/m²/d (i.e., 1120 mg/m²/cycle)
Cohort 4: 4 patients received 240 mg/m²/d (i.e., 1680 mg/m²/cycle).

The results in Table 1 show that a dose of 80 mg/m²/d in a 7-day treatment cycle, i.e., a total amount of 560 mg / m² /cycle of antisense oligonucleotide unexpectedly has the best effect in the prolongation of the survival rate of the patients, which is 13.8 months. In contrast, doses of 160 mg/m²/d or 240 mg/m²/d show a lower effect comparable with the effect of 40 mg/m²/d.

**Table 1**

| | **7-day continuous infusion, every 2 weeks** | | | |
|---|---|---|---|---|
| | **40 mg/m²/d** | **80 mg/m²/d** | **160 mg/m²/d** | **240 mg/m²/d** |
| N | 4 | 3 | 6 | 4 |
| mg/m²/cycle of antisense oligonucleotide | 280 | 560 | 1120 | 1680 |
| Number of events (death) | 4 (100.0 %) | 2 (66.7 %) | 6 (100.0 %) | 4 (100.0 %) |
| | | | | |
| Median [days] | 209 | 421 | 86 | 198 |
| (95% CI) | (33.0 - 337.0) | (139.0 - *) | (65.0 - 120.0) | (56.0 - 280.0) |
| Median [weeks] | 29.9 | 60.1 | 12.3 | 28.2 |
| Median [months] | 6.9 | 13.8 | 2.8 | 6.5 |

| | | | | |
|---|---|---|---|---|
| *An upper limit of 95 % CI for 80 mg/m²/d cannot be indicated at the moment as one patient is still alive. | | | | |

### Example 2

### Effect of an antisense oligonucleotide in a 4-day treatment cycle on pancreatic carcinoma

In another experiment, the pharmaceutical composition comprising the antisense oligonucleotide of SEQ ID NO. 22 is administered intravenously in 1 to 10 cycles, wherein one treatment cycle consists of 4 days, followed by a 10-day treatment free interval.

16 patients were enrolled in four cohorts, which were treated with increased doses of SEQ ID NO. 22:
Cohort A1: 5 patients received 140 mg/m²/d (i.e., 560 mg/m²/cycle)
Cohort A2: 3 patients received 190 mg/m²/d (i.e., 760 mg/m²/cycle)
Cohort A3: 5 patients received 250 mg/m²/d (i.e., 1000 mg/m²/cycle)
Cohort A4: 3 patients received 330 mg/m²/d (i.e., 1320 mg/m²/cycle).

Patient 1 of cohort A1 was a 65 year old man, who died 5.5 months after start of treatment, patient 2 was a 50 year old woman, who died 15.6 months after start of treatment, patient 3 was a 44 year old man, who died 4.9 months after start of treatment, and patient 5 was a 63 year old woman, who died 13.4 after start of treatment. Patient 4 of cohort A1 is still alive 14.8 months after start of treatment.

The results of cohort A2 present a lower Median Overall Survival between 5.5 and 9.3 months. Patient 1, a 53 year old woman died 3 months after start of treatment and patient 2, a 63 year old man died 9.3 months after start of treatment; patient 3 is still alive 5.5 months after treatment.

The results of the different cohorts are presented in Table 2, which show high efficiency regarding the survival rate at a dose of 140 mg/m²/d in a 4-day treatment cycle, i.e., a total amount of 560 mg / m² / cycle of the antisense oligonucleotide of SEQ ID NO. 22. This dose leads to an unexpected prolongation of the survival rate, which is 13.4 months. The dose of 140 mg/m²/d in the 4-day treatment cycle led to an prolongation of the survival rate which is comparable with the survival rate of the 80 mg/m²/d in the 7-day treatment cycle.

**Table 2**

| | **4-day contiuous infusion, followed by 10-day treatment free interval** | | | |
|---|---|---|---|---|
| | **140 mg/m²/d** | **190 mg/m²/d** | **250 mg/m²/d** | **330 mg/m²/d** |
| N | 5 | 3 | 5 | 3 |
| mg/m²/cycle of antisense oligonucleotide | 560 | 760 | 1000 | 1320 |
| Number of events (death) | 4 (80.0 %) | 3 (100.0 %) | 4 (80.0 %) | 2 (66.7 %) |
| | | | | |
| Median [days] | 409 | 284 | 298 | 92 |
| (95% CI) | (150.0 - *) | (91.0 - 346) | (84 - *) | (74 - *) |
| Median [weeks] | 58.4 | 40.6 | 42.6 | 13.1 |
| Median [months] | 13.4 | 9.3 | 9.8 | 3.0 |

| | | | | |
|---|---|---|---|---|
| *An upper limit of 95 % CI cannot be indicated as one patient is still alive. | | | | |

### Example 3

### Successful use of an antisense oligonucleotide for the preparation of a pharmaceutical composition for the treatment of a pancreatic carcinoma

A 54-year old male patient with a medical history including diabetes mellitus, arterial hypertension, lymphatic edema, teratoma of the left testis, was diagnosed with pancreatic cancer AJCC (American Joint Committee on Cancer) stage 1. Histology showed a ductal Grade 2 adenocarcinoma. The patient immediately underwent a surgical resection of the tumor (Whipple's procedure, resection grade R0) and received three chemotherapy regimens (containing 5-fluorouracil, leucovorin, and gemcitabine).

After tumor recurrence with metastatic liver metastases based on metastatic pancreatic carcinoma, the patient was treated with a pharmaceutical composition comprising the antisense oligonucleotide of SEQ ID 22 in a dose of 80 mg/m²/d over 7 days representing one cycle of treatment resulting in a total amount of 560 mg/m²/cycle. The patient underwent 7 cycles of a 7-day treatment period, wherein all the cycles were followed by a 7-day treatment free interval.

The identified liver metastases shrunk within 8 weeks from the start of the treatment and completely disappeared within 16 weeks from the beginning of the treatment. Until February 2009, 45.6 months after start of treatment with a pharmaceutical composition comprising the antisense oligonucleotide of SEQ ID No. 22, neither recurrence of primary tumor nor any new metastasis was observed on the abdominal CTs performed in periodic intervals. The patient did not receive any anti-tumor therapy after completion of the treatment with the pharmaceutical composition comprising the antisense oligonucleotide of SEQ ID No. 22. The last control CT available, which was performed in February 2009, 45.6 months after start of treatment with the pharmaceutical composition, confirmed the complete response.

### Example 4

### Median survival in pancreatic carcinoma, malignant melanoma and colorectal carcinoma

11 patients suffering from advanced pancreatic carcinoma, 2 from advanced malignant melanoma, and 4 from advanced colorectal carcinoma were treated with a pharmaceutical composition comprising the antisense oligonucleotide of SEQ ID No. 22 in a dose of either 80 mg/m²/d for 7 days (one cycle) followed by a 7-day treatment free interval or in a dose of 140 mg/m²/d for 4 days (one cycle), followed by a 10-day treatment free interval. The patients were treated for 1 to 10 cycles.

Table 3 shows the median survival in weeks and months, leading to the result that the antisense oligonucleotide of SEQ ID No. 22 in a dose of 80 mg/m²/d clearly prolonged the survival of the patients independent of the type of cancer.

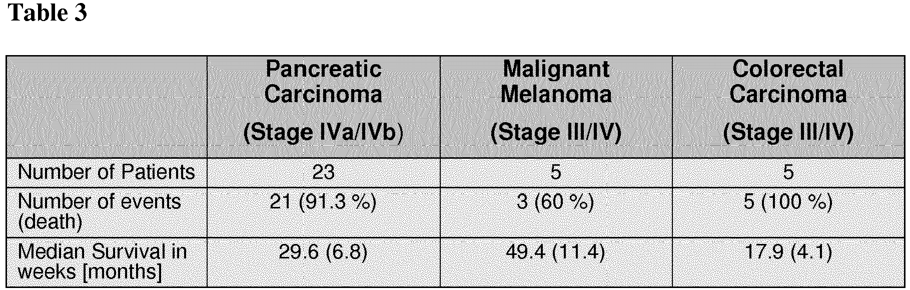

### Example 7

### Dose dependency of Cₘₐₓ in plasma

The dose dependency was measured with a validated capillary gel electrophoresis method. Plasma samples of the patients mentioned in Example 1 (Cohort A1 to A4) and Example 2 (Cohort A1 to A4) were investigated regarding the concentration of the antisense oligonucleotide of SEQ ID No. 22 (Fig. 3 and 4), wherein the antisense oligonucleotide was administered in the above mentioned doses for 7 or 4 days. The administration of the antisense oligonucleotide of SEQ ID NO. 22 in a dose of 80 mg/m²/d for 7 days (Fig. 3) or in a dose of 140 mg/m²/d for 4 days (Fig. 4), led both to a total amount of 560 mg/m²/d of the administered antisense oligonucleotide. However, the administration of the antisense oligonucleotide for 4 days (Fig. 4) surprisingly led to a higher Cₘₐₓ than the administration for 7 days (Fig. 3).

### Example 8

### Dose dependency of AUC_{0-t last} in plasma

The dose dependency was measured with a validated capillary gel electrophoresis method and subsequent pharmakokinetic analysis with the validated WinNonlin pharmakokinetic software (version 3.2, Pharsight Corp., Mountain View, California, USA). The plasma samples of the patients of Example 1 and 2 were further investigated regarding the AUC_{0-t last} of the different doses of the antisense oligonucleotide of SEQ ID NO. 22 administered for 7 or 4 days (Fig. 5 and 6). The results show that even if the administration of 80 mg/m²/d for 7 days and of 140 mg/m²/d for 4 days led to a total amount of 560 mg/m²/d of the antisense oligonucleotide, the administration of 4 days unexpectedly resulted in a higher AUC, i.e., a higher bioavailability of the antisense oligonucleotide in the plasma (Fig. 5) than the administration for 7 days (Fig. 6).

### References

Brunton, Chapter 38 in: Goodman & Gilman's The Pharmacological Basis of Therapeutics, 9th Ed., Hardman et al. Eds., McGraw-Hill, New York, 1996, pp. 934-935
Buur et al., J. Control Rel., 1990, 14, pp. 43-51
Burris H.A. et al., J. Clin. Oncol. 1997, 15(6), pp. 2403-2413
El-Hariri, L.M. et al., J. Pharm. Pharmacol., 1992, 44, pp. 651-654
Friess H. et al., Gastroenterology, 1993, 105(6), pp. 1846-1856
Kallinowski B., Recent Results Cancer Res., 2005, 165, pp. 245-249
Krieg, A.M, et al., Annu. Rev. Immunol., 2002, 20, pp. 709-760
Langer et al Science, 1990, 249, pp. 1527-1533
Lee et al., Critical Reviews in Therapeutic Drug Carrier Systems, 1991, pp. 92ff
Lefebure, C. et al., Eur. Cytokine Netw, 1995, 6, pp. 7-19
Moore M.J., J. Clin. Oncol., 2007, 25(15), pp. 1960-1966
Muranishi, Critical Reviews in Therapeutic Drug Carrier Systems, 1990, 7, pp. 1-33
Reed J.A., et al., Am. J. Pathol., 1994, 145(1), pp. 97-104
Schlingensiepen, R. et al, The Oligonucleotide, 2005, 15(2), pp. 94-104
Takahasi, H. et al., J. Pharm. Pharmacol., 1988, 40, pp. 252-257
Van Cutsem et al., J. Clin. Oncol., 2007, 25(15) pp. 1949-1952
Von Bernstoff W.M., Clin. Cancer Res., 2001, 7 (3 Suppl.), pp. 925s-932s
Wang and Martini, Clin. Invest., 1996/97, pp. 448-454
Yamashita, S. et al., J. Pharm. Pharmacol., 1987, 39, pp. 621-626
Young S.E., J. Surg. Oncol., 2006, 94(4), pp. 344-351

## Claims

1. An antisense oligonucleotide, comprising 8 to 30 nucleotide building blocks, which hybridizes with mRNA of tumor growth factor TGF-beta 2 for use in preventing and/or treating a tumor, wherein the antisense oligonucleotide is to be administered intravenously in an amount of 400 to 800 mg/m²/treatment cycle.

2. Antisense oligonucleotide for use according to claim 1, which is to be administered intravenously in an amount of 560 to 760 mg/m²/treatment cycle.

3. Antisense oligonucleotide for use according to claim 1 or 2, which is SEQ ID NO. 22.

4. Antisense oligonucleotide for use according to any of claims 1 to 3, wherein the tumor is selected from the group consisting of pancreatic carcinoma, malignant melanoma, and colorectal carcinoma.

5. Antisense oligonucleotide for use according to any of claims 1 to 4, wherein the antisense oligonucleotide is to be administered in 1, 2, 3, 4, 5, 6, 7, 8, 9, 10, 11, 12, 13, 14, or 15 cycles.

6. Antisense oligonucleotide for use according to claim 5, wherein the antisense oligonucleotide is to be administered in 1 d, 2 d, 3 d, 4 d, 5 d, 6 d, 7 d, 8 d, 9 d, 10 d, 11 d, 12 d, 13 d, 14 d, or 15 d per cycle.

7. Antisense oligonucleotide for use according to claim 6, which is to be administered on consecutive days.

8. Antisense oligonucleotide for use according to any of claims 5 to 7, wherein the time interval between the cycles is 1 d, 2 d, 3 d, 4 d, 5 d, 6 d, 7 d, 8 d, 9 d, 10 d, 11 d, 12 d, 13 d, 14 d, or 15 d.

9. Antisense oligonucleotide for use according to any of claims 1 to 8, which is to be administered in 1 to 10 cycles.

10. Antisense oligonucleotide for use according to any of claims 1 to 9, wherein the treatment cycle comprises the administration of 80 mg/m²/d for 7 days, followed by a 7-day treatment free interval or 140 mg/m²/d or 190 mg/m²/d for 4 days, followed by a 10-day treatment free interval.

11. Antisense oligonucleotide for use according to claim 10, wherein the AUC for the 4 day treatment cycle is higher than for the 7 day treatment cycle.

12. Use of an antisense oligonucleotide, comprising 8 to 30 nucleotide building blocks, which hybridizes with mRNA of tumor growth factor TGF-beta 2 for the preparation of a medicament for preventing and/or treating a tumor, wherein the antisense oligonucleotide is to be administered intravenously in an amount of 400 to 800 mg/m²/treatment cycle.

## Patentansprüche

1. Ein Antisense-Oligonukleotid, umfassend 8 bis 30 Nukleotidbausteine, die mit mRNS von Tumorwachstumsfaktor TGF-beta 2 hybridisieren, zur Verwendung bei der Vorbeugung und/oder Behandlung eines Tumors, wobei das Antisense-Oligonukleotid intravenös in einer Menge von 400 bis 800 mg/m²/Behandlungszyklus zu verabreichen ist.

2. Antisense-Oligonukleotid zur Verwendung nach Anspruch 1, welches intravenös in einer Menge von 560 bis 760 mg/m²/Behandlungszyklus zu verabreichen ist.

3. Antisense-Oligonukleotid zur Verwendung nach Anspruch 1 oder 2, welches SEQ ID NO. 22 ist.

4. Antisense-Oligonukleotid zur Verwendung nach einem der Ansprüche 1 bis 3, wobei der Tumor ausgewählt ist aus der Gruppe, bestehend aus Pankreaskarzinom, malignem Melanom, und kolorektalem Karzinom.

5. Antisense-Oligonukleotid zur Verwendung nach einem der Ansprüche 1 bis 4, wobei das Antisense-Oligonukleotid in 1, 2, 3, 4, 5, 6, 7, 8, 9, 10, 11, 12, 13, 14, oder 15 Zyklen zu verabreichen ist.

6. Antisense-Oligonukleotid zur Verwendung nach Anspruch 5, wobei das Antisense-Oligonukleotid in 1 d, 2 d, 3 d, 4 d, 5 d, 6 d, 7 d, 8 d, 9 d, 10 d, 11 d, 12 d, 13 d, 14 d, oder 15 d pro Zyklus zu verabreichen ist.

7. Antisense-Oligonukleotid zur Verwendung nach Anspruch 6, welches an aufeinanderfolgenden Tagen zu verabreichen ist.

8. Antisense-Oligonukleotid zur Verwendung nach einem der Ansprüche 5 bis 7, wobei das Zeitintervall zwischen den Zyklen 1 d, 2 d, 3 d, 4 d, 5 d, 6 d, 7 d, 8 d, 9 d, 10 d, 11 d, 12 d, 13 d, 14 d, oder 15 d beträgt.

9. Antisense-Oligonukleotid zur Verwendung nach einem der Ansprüche 1 bis 8, welches in 1 bis 10 Zyklen zu verabreichen ist.

10. Antisense-Oligonukleotid zur Verwendung nach einem der Ansprüche 1 bis 9, wobei der Behandlungszyklus die Verabreichung von 80 mg/m²/d für 7 Tage, gefolgt von einem 7-tägigen behandlungsfreien Intervall oder 140 mg/m²/d oder 190 mg/m²/d für 4 Tage, gefolgt von einem 10-tägigen behandlungsfreien Intervall, umfasst.

11. Antisense-Oligonukleotid zur Verwendung nach Anspruch 10, wobei die AUC (engl.: area under the curve; Fläche unter der Kurve) für den 4-tägigen Behandlungszyklus größer als für den 7-tägigen Behandlungszyklus ist.

12. Verwendung eines Antisense-Oligonukleotids, umfassend 8 bis 30 Nukleotidbausteine, die mit mRNS von Tumorwachstumsfaktor TGF-beta 2 hybridisieren für die Herstellung eines Medikaments zur Vorbeugung und/oder Behandlung eines Tumors, wobei das Antisense-Oligonukleotid intravenös in einer Menge von 400 bis 800 mg/m²/Behandlungszyklus zu verabreichen ist.

## Revendications

1. Oligonucléotide antisens, comprenant 8 à 30 blocs de construction de nucléotides, qui s'hybride avec l'ARNm du facteur de croissance tumorale TGF-bêta 2 pour son utilisation dans la prévention et/ou le traitement d'une tumeur, dans lequel l'oligonucléotide antisens doit être administré par voie intraveineuse dans une quantité de 400 à 800 mg/m²/cycle de traitement.

2. Oligonucléotide antisens pour son utilisation selon la revendication 1, qui doit être administré par voie intraveineuse en une quantité de 560 à 760 mg/m²/cycle de traitement.

3. Oligonucléotide antisens pour son utilisation selon la revendication 1 ou 2, qui est SEQ ID NO : 22.

4. Oligonucléotide antisens pour son utilisation selon l'une quelconque des revendications 1 à 3, dans lequel la tumeur est sélectionnée dans le groupe consistant en carcinome pancréatique, mélanome malin, et carcinome colorectal.

5. Oligonucléotide antisens pour son utilisation selon l'une quelconque des revendications 1 à 4, dans lequel l'oligonucléotide antisens doit être administré en 1, 2, 3, 4, 5, 6, 7, 8, 9, 10, 11, 12, 13, 14, ou 15 cycles.

6. Oligonucléotide antisens pour son utilisation selon la revendication 5, dans lequel l'oligonucléotide antisens doit être administré en 1 j, 2 j, 3 j, 4 j, 5 j, 6 j, 7 j, 8 j, 9 j, 10 j, 11 j, 12 j, 13 j, 14 j, ou 15 j par cycle.

7. Oligonucléotide antisens pour son utilisation selon la revendication 6, qui doit être administré des jours consécutifs.

8. Oligonucléotide antisens pour son utilisation selon l'une quelconque des revendications 5 à 7, dans lequel l'intervalle de temps entre les cycles est de 1 j, 2 j, 3 j, 4 j, 5 j, 6 j, 7 j, 8 j, 9 j, 10 j, 11 j, 12 j, 13 j, 14 j, ou 15 j.

9. Oligonucléotide antisens pour son utilisation selon l'une quelconque des revendications 1 à 8, qui doit être administré en 1 à 10 cycles.

10. Oligonucléotide antisens pour son utilisation selon l'une quelconque des revendications 1 à 9, dans lequel le cycle de traitement comprend l'administration de 80 mg/m²/j pendant 7 jours, suivi d'un intervalle sans traitement de 7 jours ou de 140 mg/m²/j ou de 190 mg/m²/j pendant 4 jours, suivi d'un intervalle sans traitement de 10 jours.

11. Oligonucléotide antisens pour son utilisation selon la revendication 10, dans lequel l'AUC pour le cycle de traitement de 4 jours est supérieure à celle pour le cycle de traitement de 7 jours.

12. Utilisation d'un oligonucléotide antisens, comprenant 8 à 30 blocs de construction de nucléotides, qui s'hybride avec l'ARNm du facteur de croissance tumorale TGF-bêta 2 pour la préparation d'un médicament destiné à la prévention et/ou au traitement d'une tumeur, dans laquelle l'oligonucléotide antisens doit être administré par voie intraveineuse dans une quantité de 400 à 800 mg/m²/cycle de traitement.
